(19) Europäisches Patentamt
European Patent Office
Office européen des brevets

(11) **EP 1 052 937 B1**

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(21) Anmeldenummer: **99963377.9**

(22) Anmeldetag: **03.12.1999**

(51) Int Cl.⁷: **A61B 6/00**, G06T 5/00

(86) Internationale Anmeldenummer:
**PCT/EP1999/009455**

(87) Internationale Veröffentlichungsnummer:
**WO 2000/033740 (15.06.2000 Gazette 2000/24)**

(54) **RÖNTGENUNTERSUCHUNGSGERÄT UND VERFAHREN ZUR ERZEUGUNG VERZERRUNGSFREIER RÖNTGENBILDER**

X-RAY EXAMINATION APPARATUS AND METHOD FOR GENERATING DISTORTION-FREE X-RAY IMAGES

APPAREIL D'EXAMEN AUX RAYONS X

(84) Benannte Vertragsstaaten:
**DE FR GB NL**

(30) Priorität: **08.12.1998 DE 19856536**

(43) Veröffentlichungstag der Anmeldung:
**22.11.2000 Patentblatt 2000/47**

(73) Patentinhaber:
• **Koninklijke Philips Electronics N.V.**
**5621 BA Eindhoven (NL)**
• **Philips Intellectual Property & Standards GmbH**
**20099 Hamburg (DE)**
Benannte Vertragsstaaten:
**DE**

(72) Erfinder:
• **ZYLKA, Waldermar**
**NL-5656 AA Eindhoven (NL)**
• **SABCZYNSKI, Jörg**
**NL-5656 AA Eindhoven (NL)**
• **WEESE, Jürgen**
**NL-5656 AA Eindhoven (NL)**

(74) Vertreter: **Volmer, Georg, Dipl.-Ing.**
**Philips Intellectual Property & Standards GmbH,**
**Postfach 50 04 42**
**52088 Aachen (DE)**

(56) Entgegenhaltungen:
EP-A- 0 623 884          DE-A- 19 703 556
FR-A- 2 631 810          US-A- 5 748 768
US-A- 5 772 594

**Beschreibung**

Die Erfindung betrifft ein Röntgenuntersuchungsgerät und Verfahren zur Erzeugung verzerrungsfreier Röntgenbilder.

Derartige Röntgenuntersuchungsgeräte und Verfahren zur Erzeugung verzerrungsfreier Röntgenbilder werden bei bestimmten chirurgischen Operationen, insbesondere in der Orthopädie benutzt, um beispielsweise die Lage von Organen und Knochen des Patienten zu betrachten. Mobile Röntgenuntersuchungsgeräte, wie z.B. C-Bogen Röntgenanordnungen, werden bei Bedarf an den Operationstisch gerollt. Die Besonderheit, die eine regelmäßige Kontrolle der Röntgenbilder auf Verzerrungen erfordert, wird durch die beweglichen Baugruppen eines solchen Röntgenuntersuchungsgerätes und die ständig veränderten Aufnahmebedingungen hervorgerufen. Auch in der Computer unterstützten Chirurgie (computer aided surgery) finden derartige Verfahren und Vorrichtungen ihre Anwendung.

In der US 5 772 594 wird ein C-Bogen-Röntgensystem beschrieben, bei dem am Bildverstärker mehrere Referenzsensoren angeordnet sind, die von einem Positionserfassungssystem geortet werden. Am zu behandelnden Knochen sind Marker angeordnet, die von diesem Positionserfassungssystem geortet werden. Bei dieser Anordnung wird das einmal aufgenommene Bild mit einer Bildverarbeitungsanordnung angezeigt und die Operationswerkzeuge des Chirurgen, die ebenfalls mit Sensoren versehen sind, werden von dem Positionserfassungssystem erfaßt und über das angezeigte Röntgenbild des abgebildeten Knochens eingeblendet. Veränderungen, die während der Operation nach Aufnahme des Bildes an der Lage des Knochens auftreten, werden nicht abgebildet.

In der EP 0 623 884 wird ebenfalls ein C-Bogen-Röntgensystem beschrieben, bei dem zum quantitativen Bestimmen der Verzerrungen von mittels eines Bildverstärkers erzeugten Röntgenaufnahmen zunächst ein verzerrtes Röntgen-Testbild eines Testobjektes angefertigt wird. Von einer Anzahl diskreter Bildpunkte werden in dem Testbild Koordinaten gemessen, die innerhalb des Testobjektes Objektpunkten mit definierter Lage und von ihrer Umgebung deutlich abweichende Absorption zugeordnet sind. Weiterhin werden die Koordinaten berechnet, mit den gemessenen verglichen und daraus eine, zum Entzerren von Röntgenbildern verwendete Transformationsfunktion ermittelt. Ein ähnliches Röntgensystem ist aus der US 5 748 768 bekannt. Der Unterschied besteht lediglich in der Generierung der Transformationsfunktion, die dann in Form einer Korrekturtabelle für jeden Bildpunkt abgelegt wird.

Bilder, die von Röntgenuntersuchungsgeräten geliefert werden, sind in der Regel verzerrt. Diese Verzerrungen entstehen einerseits durch die gekrümmte Oberfläche des Bildverstärkers und andererseits durch Veränderungen des äußeren Magnetfeldes. Außerdem wird gerade bei C-Bogen-Röntgenuntersuchungsgeräten dieses durch das Gewicht des Bildverstärkers und des Röntgenstrahlers Verbiegungen ausgesetzt, die nicht konstant sind. Derartige Verbiegungen und Verzerrungen führen zu Änderungen der Abbildungseigenschaften und damit zu fehlerhaften Röntgenaufnahmen. Mobile C-Bögen können vom Chirurgen jederzeit an eine andere Stelle oder in eine andere Ausrichtung bewegt werden, wodurch die Abbildungseigenschaften ständig verändert werden.

Für neuartige Operationstechniken, wie CAS (Computer Aided Surgerey), ist es notwendig die vollständigen Abbildungseigenschaften des Röntgenuntersuchungsgerätes zu kennen.

Aufgabe der Erfindung ist es deshalb, ein Verfahren und eine Anordnung anzugeben, mit denen die Bestimmung der Abbildungseigenschaften des Röntgenuntersuchungsgeräte aus den Patientenaufnahmen möglich ist.

Erfindungsgemäß wird diese Aufgabe dadurch gelöst, daß das Röntgenuntersuchungsgerät wenigstens einen im Röntgenstrahlengang angeordneten und in einem Referenzbild abgebildeten Kalibrationskörper und eine Korrektureinheit, die zur Korrektur von Verzerrungen in Röntgenbildern mittels Berechnung von Abbildungseigenschaften des Röntgenuntersuchungsgerätes anhand von Unterschieden zwischen den Positionen der Abbildungen der Kalibrationskörper in einer Patientenröntgenaufnahme und im Referenzbild vorgesehen ist, aufweist.

Die Kalibrationskörper sind am Gehäuse des Röntgenstrahlers und/oder am Gehäuse des Bildverstärkers angebracht und werden somit bei jeder Röntgenaufnahme abgebildet. In einer Referenzausrichtung wird ein Referenzbild aufgenommen. In diesem Referenzbild werden die Positionen der im Referenzbild abgebildeten Kalibrationskörper bestimmt und gespeichert. Mit Hilfe der bekannten Geometrie der Kalibrationskörper und der bekannten Position und Orientierung relativ zum Bildverstärker bzw. Röntgenstrahler werden die Abbildungseigenschaften des Röntgenuntersuchungsgerätes bestimmt.

Bei Aufnahme des Patienten kann das Röntgenuntersuchungsgerät eine andere Ausrichtung haben oder es können andere, die Patientenröntgenaufnahme verfälschende, Einflüsse wirken, wie bei Aufnahme des Referenzbildes. Die Positionen der Abbildungen der Kalibrationskörper in der Patientenaufnahme werden in der Korrektureinheit mit den gespeicherten Positionen der Kalibrationskörper im Referenzbild verglichen. Aus sich ergebenden Unterschieden oder Differenzen zwischen Positionen der Kalibrationskörper in der Patientenaufnahme und den Positionen im Referenzbild werden Verzerrungen in der Patientenröntgenaufnahme berechnet und korrigiert, so daß ein verzerrungsfreies Röntgenbild von der Ausgabeeinheit ausgegeben wird.

Die Abbildungseigenschaften von Röntgenuntersuchungsgeräten können mit Hilfe von Referenzbildern von Objekten mit bekannter Geometrie, sogenannten Kalibrationskörpern, bestimmt werden. Sind diese Abbildungseigenschaften des Röntgenuntersuchungsgerätes bekannt, können Aufnahmen anderer Objekte, z.B. Patientenröntgenaufnahmen korrigiert werden.

Einer Berechnungseinheit wird das Referenzbild zugeführt. Aus den Positionen der Abbildungen der Kalibrationskörper in diesem Referenzbild und den bekannten Abmessungen des Röntgenuntersuchungsgerätes werden in der Berechnungseinheit die Referenzabbildungseigenschaften berechnet und in einem Speicher gespeichert.

Eine weitere Ausführungsform der Erfindung sieht vor, am Bildverstärker und/oder Röntgenstrahler Bezugsmarken anzubringen. Diese Bezugsmarken werden von einer Positionserfassungsvorrichtung geortet. Die Positionserfassungsvorrichtung definiert ein Koordinatensystem, in dem die Positionen der Bezugsmarken erfaßt werden. Die erfaßten Positionen werden der Berechnungseinheit zugeführt. Aus den Abständen zwischen Bezugsmarken und Kalibrationskörpern am Bildverstärkers und Röntgenstrahlers und deren bekannten Geometrie bezüglich der Bezugsmarken werden die Referenzabbildungseigenschaften des Röntgenuntersuchungsgerätes bestimmt.

Ein Kalibrationsphantom, welches eine andere Form als die Kalibrationskörper aufweisen kann, wird in den Röntgenstrahlengang, beispielsweise am Patienten oder Operationstisch, eingebracht. An diesem Kalibrationsphantom sind Bezugsmarken angeordnet. Diese Bezugsmarken werden ebenfalls von der Positionserfassungsanordnung erfaßt und zur Berechnungseinheit übertragen. Durch die bekannte geometrische Form des Kalibrationsphantoms und den Positionen der Bezugsmarken wird die Position des Kalibrationsphantoms in dem Koordinatensystem bestimmt. Aus der bekannten Position des Kalibrationsphantoms im Koordinatensystem mit den vorher berechneten Referenzabbildungseigenschaften wird die Position des Kalibrationsphantoms in der zu erstellenden Patientenröntgenaufnahme berechnet. Die berechnete Position des Kalibrationsphantoms wird mit der tatsächlichen Position des Kalibrationsphantoms in der Patientenröntgenaufnahme verglichen. Aus auftretenden Unterschieden werden die Abbildungseigenschaften des Röntgenuntersuchungsgerätes bei Aufnahme der Patientenröntgenaufnahme berechnet und Verzerrungen in der Patientenröntgenaufnahme gegebenenfalls in der Korrektureinheit korrigiert.

Als vorteilhaft erweist es sich die Kalibrationskörper in einer für Röntgenstrahlen durchsichtigen Scheibe kreisförmig anzuordnen. Dadurch ist es möglich, die Kalibrationskörper am Rand der Patientenröntgenaufnahme abzubilden und das Röntgenbild sowenig wie möglich zu beeinflussen. Die Kalibrationskörper bestehen vorteilhafterweise aus Metallkugeln, die die Röntgenstrahlen absorbieren und deshalb in der Röntgenaufnahme sichtbar sind.

In einer weiteren Ausführungsform sind die Kalibrationskörper in einer durchsichtigen Scheibe als gekreuzte Metallfäden angeordnet. Auch hier ergibt sich im Fokus des Röntgenstrahlengangs ein freier Bereich, der nicht von den Kalibrationskörpern überlagert wird. Außerdem lassen sich mit den gekreuzten Metallfäden schon mit bloßem Auge Verzerrungen feststellen, da die normalerweise geraden Metallfäden im Referenzbild bei Verzerrungen in der Patientenröntgenaufnahme durchgebogen sind.

Konstruktionsbedingt erweist es sich als vorteilhaft mit Bezugsmarken versehene Kalibrationsphantome direkt am Patienten oder am Operationstisch anzubringen. Die Position der Bezugsmarken ist von der Positionserfassungsanordnung ortbar. Bei den unterschiedlichen Ausrichtungen des Röntgenuntersuchungsgerätes ist es möglich, daß einige Bezugsmarken verdeckt werden, so daß sie von der Positionserfassungsanordnung nicht geortet werden können. In diesem Fall erweist es sich als vorteilhaft, auf Kalibrationskörper oder -phantome zurückzugreifen, bei denen die Bezugsmarken von der Positionserfassungsanordnung geortet werden können.

Die Aufgabe wird auch durch ein Verfahren zur Erzeugung von verzerrungsfreien Röntgenbildern gelöst, bei dem mit einem Röntgenuntersuchungsgerät mit Röntgenstrahler, Bildverstärker und Kalibrationskörpern, Referenzbilder in Referenzausrichtungen des Röntgenuntersuchungsgerätes mit am Bildverstärker oder Röntgenstrahler angeordneten und abgebildeten Kalibrationskörpern aufgenommen werden und die Positionen der in einer Patientenröntgenaufnahme abgebildeten Kalibrationskörper mit den Positionen der Kalibrationskörper im entsprechenden Referenzbild verglichen werden und aus Unterschieden auftretende Verzerrungen im Röntgenbild korrigiert werden.

Eine weitere Ausführungsform der Erfindung sieht vor, mehrere Referenzbilder aufzunehmen und für diese Referenzbilder jeweils die Referenzabbildungseigenschaften zu bestimmen. Für den Vergleich der Positionen der Kalibrationskörper in den Patientenröntgenaufnahmen wird das Referenzbild ausgewählt, welches in seiner Ausrichtung der Ausrichtung des Röntgenuntersuchungsgerätes bei Aufnahme der Patientenröntgenaufnahme am ähnlichsten ist. Die Positionen der Kalibrationskörper in diesem geeigneten Referenzbild werden mit den Positionen der Kalibrationskörper in der Patientenröntgenaufnahme verglichen und aus sich ergebenden Unterschieden die Abbildungseigenschaften berechnet und ggf. Verzerrungen in der Darstellung der Patientenröntgenaufnahme korrigiert.

Als vorteilhaft erweist es sich, mehrere Referenzbilder in unterschiedlichen Ausrichtungen aufzunehmen. Für diese unterschiedlichen Ausrichtungen werden jeweils die Referenzabbildungseigenschaften berechnet und in einem Speicher gespeichert.

Die ausrichtungsabhängigen Referenzabbildungseigenschaften werden in einer Look-Up Table gespeichert und setzen sich aus mehreren relevanten Daten zusammen, beispielsweise die relative Position des Röntgenstrahlers bezüglich des Bildverstärkers und die Position des Röntgenuntersuchungsgerätes relativ zu einem äußeren Referenzkörper, z. B. dem Patienten. Mit Hilfe einer Interpolation lassen sich somit die Referenzabbildungseigenschaften für alle möglichen Ausrichtungen des Röntgenuntersuchungsgerätes berechnen. Für den Vergleich der Positionen der Kalibrationskörper werden als Referenz die Daten in der Look-Up Table verwendet, die dann mit den tatsächlichen Positionen der Abbildung der Kalibrationskörper in der Patientenröntgenaufnahme verglichen werden. Aus sich ergebenden Unterschieden oder Differenzen werden Verzerrungen in der Patientenröntgenaufnahme korrigiert. Diese Ausführungsform erhöht die Genauigkeit der Berechnung der Abbildungseigenschaften, da jede Ausrichtung des Röntgenuntersuchungsgerätes bei Aufnahme der Patientenröntgenaufnahme nachgebildet werden kann.

Mit dem erfindungsgemäßen Röntgenuntersuchungsgerät und dem Verfahren ist möglich, mit einem vorher aufgenommenen Referenzbild und daraus ermittelten Abbildungseigenschaften Verzerrungen in den Patientenröntgenaufnahmen zu entfernen. Der zu untersuchende Patient wird keiner zusätzlichen Röntgendosis ausgesetzt. Speziell für mobile Röntgenuntersuchungsgeräte, bei denen sich die Aufnahmebedingungen häufig stark ändern, ist es notwendig, die Abbildungseigenschaften zu kennen, um die aufgenommenen Röntgenbilder zu entzerren.

Auf dem Gebiet der chirurgischen Navigation besteht zudem die Möglichkeit, bei bekannten Abbildungseigenschaften des Röntgenuntersuchungsgerätes die Position und Lage von chirurgischen Werkzeugen in die entzerrten Patientenröntgenaufnahmen einzublenden.

Vorzugsweise wird als Röntgenuntersuchungsgerät ein C-Bogen Röntgengerät verwendet. Prinzipiell kann ein erfindungsgemäßes Röntgenuntersuchungsgerät und Verfahren auch im Bereich der Tomosynthese und der Computertomographie angewendet werden, da es auch für derartige Anwendungen für die Rekonstruktion oder Entzerrung der Patientenröntgenaufnahmen notwendig ist, die Abbildungseigenschaften der jeweiligen Röntgengeräte genau zu kennen.

Nachfolgend wird ein Ausführungsbeispiel anhand der Zeichnung näher erläutert.

Fig. 1     eine schematische Darstellung einer C-Bogen Röntgenanordnung,
Fig. 2     Kalibrationskörper des Bildverstärkers,
Fig. 3     Kalibrationskörper am Röntgenstrahler,
Fig. 4     das Referenzbild mit Kalibrationskörpern,
Fig. 5     verzerrte Patientenröntgenaufnahme,
Fig. 6     entzerrte Patientenröntgenaufnahme.

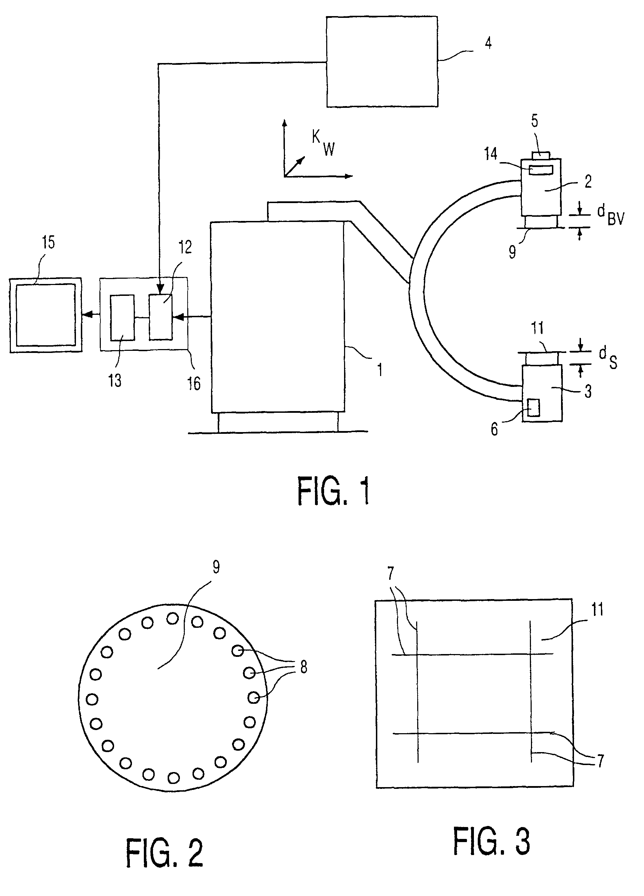

In Fig. 1 ist am Gehäuse 1 einer C-Bogen Röntgenanordnung ein Bildverstärker 2 und ein Röntgenstrahler 3 angeordnet. Am Bildverstärker 2 sind in einem Abstand $d_{BV}$ Kalibrationskörper 8 in einer Scheibe 9 angebracht. Gleichzeitig befindet sich im Bildverstärker 2 eine CCD-Kamera 14, mit der die Patientenröntgenaufnahme aufgenommen wird und an die Bildverarbeitungseinheit 16 weitergeleitet wird. Am Bildverstärker 2 sind Bezugsmarken 5 angebracht. Diese Bezugsmarken 5 können bei optischen Positionserfassungsanordnungen 4 mit LED's realisiert sein, sie können aber auch einfache geometrische Körper sein, die eindeutige Formen aufweisen, die von einer Positionserfassungsanordnung 4 geortet und unterschieden werden können. Bei magnetischen Positionserfassungsanordnungen könnten diese Bezugsmarken durch magnetische Sensoren realisiert sein. Auch auf Funk oder Infrarot basierende Positionserfassungsvorrichtungen sind einsetzbar.

Auf der gegenüberliegenden Seite des Bildverstärkers 2 ist der Röntgenstrahler 3 angeordnet, der die Röntgenstrahlung aussendet, die dann vom Bildverstärker aufgenommen wird und dort in sichtbares Licht gewandelt wird, welches von der CCD-Kamera 14 aufgenommen wird und zur Bildverarbeitungsanlage 16 weitergeleitet wird. Der Röntgenstrahler 3 weist ebenso Bezugsmarken 6 auf, die von der Positionserfassungsanordnung 4 geortet werden. Vor dem Röntgenstrahler 3 ist in einem Abstand ds eine durchsichtige Scheibe 11 mit den Kalibrationskörpern 7 in Form von gekreuzten Metallfäden angeordnet. In der Bildverarbeitungseinheit 16 befindet sich eine Berechnungseinheit 12 und eine Korrektureinheit 13. Die entzerrte Patientenröntgenaufnahme wird von der Ausgabeeinheit 15 ausgegeben.

Es wird ein Referenzbild aufgenommen, in dem die Kalibrationskörper 7,8 abgebildet sind. Das Referenzbild mit den abgebildeten Kalibrationskörpern kann sowohl schon nach der Herstellung des Röntgenuntersuchungsgerätes, als auch direkt vor der Operation aufgenommen werden. Es wird der Berechnungseinheit 12 zugeführt. Die Berechnungseinheit 12 bestimmt die Positionen der Kalibrationskörper im Referenzbild und berechnet anhand der bekannten Geometrie des C-Bogen Röntgengerätes die Referenzabbildungseigenschaften. Diese werden in einem Speicher, der hier nicht dargestellt ist, gespeichert.

Zusätzlich sind die Positionen des Bildverstärkers 2 und des Röntgenstrahlers 3 durch Ortung der Bezugsmarken 5 und 6 von der Positionserfassungsanordnung 4 in einem räumlichen Koordinatensystem $K_w$ in ihrer Lage bekannt.

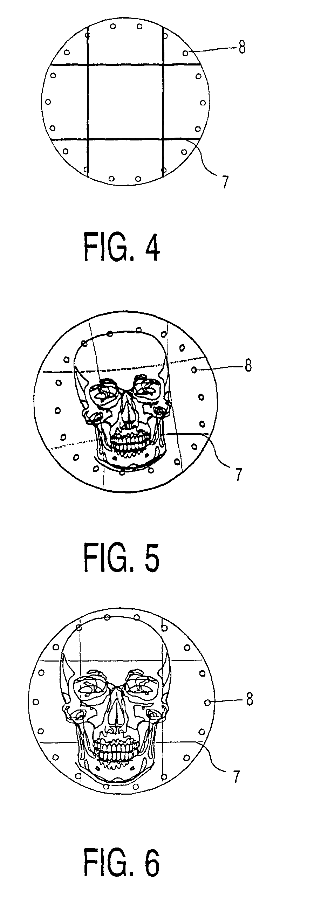

In der Berechnungseinheit 12 werden die gespeicherten Positionen der Kalibrationskörper 7 und 8 in Form der gekreuzten Fäden 7 und der kreisförmig angeordneten Metallkugeln 8 aus dem Referenzbild (Fig.4) mit den Positionen der Abbildungen der Kalibrationskörper in der Patientenröntgenaufnahme(Fig.5) verglichen.

Nach Aufnahme der Patientenröntgenaufnahme in einer anderen Ausrichtung des C-Bogens kann eine verzerrte Patientenröntgenaufnahme (Fig. 5) auftreten. Durch diese Verzerrungen in der Patientenröntgenaufnahme entstehen Unterschiede oder Differenzen zwischen den Positionen der Kalibrationskörper. Mit diesen Differenzen und den Referenzabbildungseigenschaften werden dann die Änderungen der Abbildungseigenschaften für die C-Bogen Röntgenanordnung berechnet. Anhand dieser Berechnung wird die Korrektur von Verzerrungen in der Patientenröntgenaufnahme in der Korrektureinheit 13 vorgenommen. Die entzerrte Patientenröntgenaufnahme (Fig. 6) wird mittels der Ausgabeeinheit 15 beispielsweise auf einem Monitor angezeigt.

Figur 2 und 3 zeigen die Scheiben 9 und 11 in den jeweils die Kalibrationskörper 7 und 8 angeordnet sind. Die Kalibrationskörper sind jeweils am Rand der Scheibe angeordnet um das Feld für die Aufnahme des Patienten und damit die Betrachtung der Patientenröntgenaufnahme durch den Chirurgen so wenig wie möglich zu stören.

Im folgenden soll die Berechnung der Differenzen der Positionen der Kalibrationskörper in den Patientenröntgenaufnahmen und in den Referenzbildern kurz dargestellt werden. Sei $p$ ein beliebiger Parameter des Modells, das die Abbildungseigenschaften der C-Bogen Röntgenanordnung beschreibt. Seien $B_R$ das Referenzbild und $B_P$ das Patientenbild und $K(B)= \{k_i(B)\}$ der Satz aller Positionen der Kalibrationskörper $k_i(B)$ im Bild B. Der Schätzwert des Parameters $\bar{p}$ ist eine Funktion von K(B). Damit läßt sich $\bar{p}$ in eine Taylorreihe nach den Differenzen der Lage der Kalibrationskörper im Patientenbild und im Referenzbild entwickeln:

$$\bar{p}(K(B_P))=\bar{p}(K(B_R))+c_1(K(B_P)-K(B_R))+c_2(K(B_P)-K(B_R))^2 + ....$$

Diese Gleichung ist in vereinfachter Form dargestellt, die wirkliche Gleichung ist komplexer. Die Konstanten $c_1$, $c_2$ ... ergeben sich aus dem Abbildungsmodell. Einige Parameter des Abbildungsmodells verändern sich nicht oder nur unwesentlich und können mit dem Referenzbild gemessen und zum Patientenbild unverändert übernommen werden. Welche Parameter dies sind, hängt von der jeweiligen C-Bogen Röntgenanordnung ab und kann entweder durch Messungen oder durch Modellrechnungen ermittelt werden. Mit der Verbiegung der C-Bogen Röntgenanordnung ist z.B. auch eine Veränderung der relativen Orientierung (Rotation) des Bildverstärkers zum Röntgenstrahler verbunden. Dieser Effekt kann jedoch meistens gegenüber der Veränderung der relativen Position (Translation) der beiden Gehäuse zueinander vernachlässigt werden. Um die drei Parameter der Translation zu bestimmen, reichen schon wenige Kalibrationskörper im Patientenbild aus. Die Zahl der Kalibrationskörper im Patientenbild kann somit deutlich geringer sein, als die Zahl der Kalibrationskörper, die im Referenzbild zur Bestimmung aller Parameter des Abbildungsmodells benötigt wird.

Mit einer Vielzahl von Referenzbildern und dazu berechneten Referenzabbildungseigenschaften läßt sich der Rechenaufwand reduzieren. Da die Patientenröntgenaufnahme meist in einer anderen Ausrichtung als das Referenzbild aufgenommen wird, kann mit Referenzbildern, die in typischen, häufig vorkommenden Ausrichtungen aufgenommen wurden, die Zahl der zu berechnenden Parameter reduziert werden, indem das Referenzbild mit der Ausrichtung zur Berechnung der Abbildungseigenschaften ausgewählt wird, welches der Ausrichtung bei Aufnahme der Patientenröntgenaufnahme am ähnlichsten ist.

Ein nichtdargestelltes Kalibrationsphantom ist mit Bezugsmarken versehen und wird in den Röntgenstrahlengang eingebracht und ist beispielsweise am Patienten oder am Operationstisch befestigt. Da es genau wie Bildverstärker und Röntgenstrahler Bezugsmarken aufweist, ist es von der Positionserfassungsanordnung ortbar. Damit läßt es sich mit seinen Positionen und Abmessungen in dem Koordinatensystem definieren. Die Lage des Kalibrationsphantoms wird für die Patientenröntgenaufnahme berechnet und mit den Positionen und Abbildungen des Kalibrationsphantoms in der tatsächlichen Patientenröntgenaufnahme verglichen. Auch damit lassen sich die Abbildungseigenschaften des C-Bogen bestimmen.

Das Kalibrationsphantom kann auch ein Operationswerkzeug sein, welches mit Bezugsmarken versehen ist. Dann kann es mit den korrigierten Abbildungseigenschaften auf dem Monitor dargestellt werden, ohne das eine weitere Röntgenaufnahme notwendig ist.

Bei der Computertomographie (CT) wird aus mehreren Schnittbildern ein dreidimensionales Röntgenbild rekonstruiert. Da auch hier äußerer Magnetfelder auf das CT-Gerät wirken und Verschiebungen zwischen Röntgenstrahler und Detektor die Patientenröntgenaufnahme negativ beeinflussen, ist für die Rekonstruktion eines verzerrungsfreien Röntgenbildes die Kenntnis der Abbildungseigenschaften des CT-Gerätes notwendig. Die Kalibrationskörper werden in diesem Fall so angeordnet, daß sie in jedem Schnittbild sichtbar sind und mittels der Unterschiede zwischen den im Referenzbild abgebildeten Positionen der Kalibrationskörper und den in der Patientenröntgenaufnahme abgebildeten Positionen der Kalibrationskörper die Abbildungseigenschaften des CT-Gerätes ermittelt werden können.

Für die Tomosynthese, bei der ebenfalls Schnittbilder aufgenommen werden, ist durch den Einsatz von Kalibrationskörpern und der Feststellung der Abbildungseigenschaften die erfindungsgemäße Korrektur von Verzerrungen möglich.

**Patentansprüche**

1.  Röntgenuntersuchungsgerät mit wenigstens einem im Röntgenstrahlengang angeordneten und in einem Referenzbild (Fig.4) abgebildeten Kalibrationskörper (7,8) und einer Korrektureinheit (13), die zur Korrektur von Verzerrungen in Röntgenbildern mittels Berechnung von Abbildungseigenschaften des Röntgenuntersuchungsgerätes anhand von Unterschieden zwischen den Positionen der Abbildungen der Kalibrationskörper (7,8) in einer Patientenröntgenaufnahme (Fig.5) und im Referenzbild (Fig.4) vorgesehen ist.

2.  Röntgenuntersuchungsgerät nach Anspruch 1, **dadurch gekennzeichnet, daß** eine Berechnungseinheit (12) zur Berechnung von Referenzabbildungseigenschaften aus bekannten Abmessungen des Röntgenuntersuchungsgerätes und den Positionen der Kalibrationskörper (7,8) in dem in einer Referenzausrichtung aufgenommenen Referenzbild (Fig.4) und eine Anzeigeeinheit (15) zur Darstellung des verzerrungsfreien Röntgenbildes (Fig.6) vorgesehen sind.

3.  Röntgenuntersuchungsgerät nach Anspruch 1 und 2, **dadurch gekennzeichnet, daß** wenigstens eine an einem Bildverstärker (2) und/oder Röntgenstrahler (3) angeordnete Bezugsmarke (5,6) vorgesehen ist und ein mit weiteren Bezugsmarken versehenes, im Röntgenstrahlengang angeordnetes Kalibrationsphantom und eine Positionserfassungsvorrichtung (4) zur Erfassung der Positionen der Bezugsmarken (5,6) innerhalb eines von der Positionserfassungsvorrichtung (4) gebildeten ortsfesten Koordinatensystems $K_w$ und die Berechnungseinheit (12) zur Zuführung der erfaßten Positionen vorgesehen sind.

4.  Röntgenuntersuchungsgerät nach Anspruch 1 bis 3, **dadurch gekennzeichnet, daß** ein Speicher zur Speicherung von mehreren Referenzabbildungseigenschaften vorgesehen ist.

5.  Röntgenuntersuchungsgerät nach den Ansprüchen 1 bis 4, **dadurch gekennzeichnet, daß** wenigstens ein Kalibrationskörper (7,8) in einer im Röntgenstrahlengang angeordneten, für Röntgenstrahlung durchsichtigen Scheibe (9) angeordnet ist.

6.  Röntgenuntersuchungsgerät nach den Ansprüchen 1 bis 5, **dadurch gekennzeichnet, daß** Kalibrationskörper aus Röntgenstrahlen absorbierenden Körpern gebildet sind.

7.  Verfahren zur Erzeugung eines verzerrungsfreien Röntgenbildes mit einem Röntgenuntersuchungsgerät mit Röntgenstrahler (3), Bildverstärker (2), und Kalibrationskörpern (7,8), **dadurch gekennzeichnet, daß** Referenzbilder (Fig.4) in Referenzausrichtungen des Röntgenuntersuchungsgerätes mit am Bildverstärker (2) oder Röntgenstrahler (3) angeordneten und abgebildeten Kalibrationskörpern (7,8) aufgenommen werden und daß die Positionen der in einer Patientenröntgenaufnahme (Fig.5) abgebildeten Kalibrationskörper (7,8) mit den Positionen der Kalibrationskörper (7,8) im entsprechenden Referenzbild (Fig.4) verglichen werden und aus Unterschieden auftretende Verzerrungen im Röntgenbild korrigiert werden.

8.  Verfahren zur Erzeugung eines verzerrungsfreien Röntgenbildes nach Anspruch 7, **dadurch gekennzeichnet, daß** aus mehreren in unterschiedlichen Ausrichtungen aufgenommenen Referenzbildern (Fig.4) und daraus berechneten Referenzabbildungseigenschaften das Referenzbild (Fig.4) mit der Ausrichtung ausgewählt wird, die der Ausrichtung des Röntgenuntersuchungsgerätes bei Aufnahme der Patientenröntgenaufnahme (Fig.5) am ähnlichsten ist, um die Positionen der Kalibrationskörper (7,8) in der Patientenröntgenaufnahme (Fig.5) und in dem ausgewählten Referenzbild (Fig.4) zu vergleichen und damit Abbildungseigenschaften zu berechnen und gegebenenfalls Korrekturen in der Patientenröntgenaufnahme vorzunehmen.

9.  Verfahren nach Anspruch 7 und 8, **dadurch gekennzeichnet, daß** aus in mehreren Ausrichtungen aufgenommenen Referenzbildern (Fig.4) Referenzabbildungseigenschaften berechnet und mittels einer Interpolation die Referenzabbildungseigenschaften für alle möglichen Ausrichtungen des Röntgenuntersuchungsgerätes berechnet und insbesondere in einer Look Up Table gespeichert werden.

**Claims**

1.  An X-ray examination apparatus which includes at least one calibration member (7, 8) which is arranged in the X-ray beam path and is reproduced in a reference image (Fig. 4), and a correction unit (13) which is arranged to correct distortions in X-ray images by calculating imaging properties of the X-ray examination apparatus on the basis of differences between the positions of the reproductions of the calibration members (7, 8) in a patient X-ray image (Fig. 5) and in the reference image (Fig. 4).

**2.** An X-ray examination apparatus as claimed in claim 1, **characterized in that** there are provided an arithmetic unit (12) for calculating reference imaging properties from known dimensions of the X-ray examination apparatus and the positions of the calibration members (7, 8) in the reference image (Fig. 4) acquired in a reference orientation, and a display unit (15) for displaying the distortion-free X-ray image (Fig. 6).

**3.** An X-ray examination apparatus as claimed in the claims 1 and 2, **characterized in that** there are provided at least one reference marker (5, 6) which is mounted on an image intensifier (2) and/or an X-ray source (3), and also a calibration phantom which is provided with further reference markers and is arranged in the X-ray beam path, and a position measuring device (4) for measuring the positions of the reference markers (5, 6) in a fixed co-ordinate system $K_W$ formed by the position measuring device (4), and the arithmetic unit (12) for supplying the measured positions.

**4.** An X-ray examination apparatus as claimed in the claims 1 to 3, **characterized in that** a memory is provided for the storage of a plurality of reference imaging properties.

**5.** An X-ray examination apparatus as claimed in the claims 1 to 4, **characterized in that** at least one calibration member (7, 8) is provided in a disc (9) which is arranged in the X-ray beam path and is transparent to X-rays.

**6.** An X-ray examination apparatus as claimed in the claims 1 to 5, **characterized in that** calibration members consist of X-ray absorbing members.

**7.** A method of forming a distortion-free X-ray image by means of an X-ray examination apparatus which includes an X-ray source (3), an image intensifier (2) and calibration members (7, 8), **characterized in that** reference images (Fig. 4) are formed in reference orientations of the X-ray examination apparatus with calibration members which are mounted on the image intensifier (2) or the X-ray source (3) and are reproduced, and that the positions of the calibration members (7, 8) reproduced in a patient X-ray image (Fig. 5) are compared with the positions of the calibration members (7, 8) in the corresponding reference image (Fig. 4), and that distortions occurring in the X-ray image which are due to differences are corrected.

**8.** A method of forming a distortion-free X-ray image as claimed in claim 7, **characterized in that** from a plurality of reference images (Fig. 4) formed in different orientations and from reference imaging properties calculated therefrom that reference image (Fig. 4) is selected whose orientation is most similar to the orientation of the X-ray examination apparatus during the acquisition of the patient X-ray image (Fig. 5) in order to compare the positions of the calibration members (7, 8) in the patient X-ray image (Fig. 5) and in the selected reference image (Fig. 4) so as to calculate imaging properties on the basis thereof and to carry out, if necessary, corrections in the patient X-ray image.

**9.** A method as claimed in the claims 7 and 8, **characterized in that** reference imaging properties are calculated from reference images (Fig. 4) formed in a plurality of orientations, which properties are calculated, by way of interpolation, for all feasible orientations of the examination apparatus and are stored notably in a look-up table.

**Revendications**

**1.** Appareil d'examen aux rayons X avec au moins un corps de calibrage (7,8) disposé dans le trajet des rayons X et représenté dans une image de référence (Fig. 4) et une unité de correction (13) qui est prévue pour la correction des distorsions dans les images radiographiques par le calcul des propriétés de représentation de l'appareil d'examen aux rayons X à l'aide de différences entre les positions des représentations des corps de calibrage (7,8) dans un cliché radiographique du patient (Fig. 5) et dans l'image de référence (Fig. 4).

**2.** Appareil d'examen aux rayons X selon la revendication 1, **caractérisé en ce qu'**une unité de calcul (12) est prévue pour le calcul des propriétés de représentation de référence à partir des dimensions connues de l'appareil d'examen aux rayons X et des positions des corps de calibrage (7, 8) dans l'image de référence enregistrée dans une orientation de référence (Fig. 4) et une unité d'affichage (15) pour la représentation de l'image radiographique sans distorsion (Fig. 6).

**3.** Appareil d'examen radiographique selon l'une des revendications 1 et 2, **caractérisé en ce qu'**au moins une marque de référence (5, 6) disposée sur un amplificateur d'image (2) et/ou un générateur de rayons X (3) est prévue, de même qu'un fantôme de calibrage doté de marques de référence supplémentaires et disposé dans le trajet de rayons X et un dispositif d'enregistrement de position (4) pour l'enregistrement des marques de référence (5, 6) à l'intérieur d'un système de coordonnées $K_w$ stationnaire formé par le dispositif d'enregistrement de position (4) et l'unité de calcul (12) pour amener les positions enregistrées.

**4.** Appareil d'examen aux rayons X selon l'une des revendications 1 à 3, **caractérisé en ce qu'**une mémoire est prévue pour l'enregistrement de plusieurs propriétés de représentation de référence.

**5.** Appareil d'examen aux rayons X selon l'une des revendications 1 à 4, **caractérisé en ce qu'**au moins un corps de calibrage (7, 8) est disposé dans un disque (9) transparent aux rayons X et disposé dans le trajet des rayons X.

**6.** Appareil d'examen aux rayons X selon l'une des revendications 1 à 5, **caractérisé en ce que** les corps de calibrage sont formés de corps absorbant les rayons X.

**7.** Procédé de production d'une image radiographique sans distorsion avec un appareil d'examen aux rayons X doté d'un générateur de rayons X (3), d'un amplificateur d'image 2 et de corps de calibrage (7,8), **caractérisé en ce que** des images de référence (Fig. 4) sont enregistrées dans des orientations de référence de l'appareil d'examen aux rayons X avec des corps de calibrage (7,8) représentés et disposés sur l'amplificateur d'image (2) ou le générateur de rayons X (3) et que les positions des corps de calibrage (7, 8) représentés dans un cliché radiographique du patient (Fig. 5) sont comparées aux positions des corps de calibrage (7, 8) dans l'image de référence correspondante (Fig. 4) et corrigées à partir de distorsions résultant de différences dans l'image photographique.

**8.** Procédé de production d'une image radiographique sans distorsion selon la revendication 7, **caractérisé en ce qu'**à partir de plusieurs images de référence (Fig. 4) enregistrées dans différentes orientations et des propriétés de représentation de référence calculées à partir de celles-ci, l'image de référence (Fig. 4) est sélectionnée avec l'orientation qui est la plus proche de l'orientation de l'appareil d'examen aux rayons X en cas d'enregistrement du cliché radiographique du patient (Fig. 5) afin de comparer les positions des corps de calibrage (7, 8) dans le cliché radiographique du patient (Fig. 5) et dans l'image de référence (Fig. 4) sélectionnée et calculer ainsi des propriétés de représentation et, éventuellement, d'apporter des corrections dans le cliché radiographique du patient.

**9.** Procédé selon l'une des revendications 7 et 8, **caractérisé en ce que** les propriétés de représentation de référence sont calculées à partir d'images de référence (Fig. 4) enregistrées dans plusieurs orientations et les propriétés de représentation de référence sont calculées par interpolation pour toutes les orientations possibles de l'appareil d'examen aux rayons X et, en particulier, enregistrées dans une Look-Up Table.

FIG. 1

FIG. 2

FIG. 3

FIG. 4

FIG. 5

FIG. 6